## Europäisches Patentamt
## European Patent Office
## Office européen des brevets

(19)

(11) Veröffentlichungsnummer: **0 136 363**
**A1**

(12) ## EUROPÄISCHE PATENTANMELDUNG

(21) Anmeldenummer: **83108821.6**

(22) Anmeldetag: **07.09.83**

(51) Int. Cl.⁴: **A 61 N 1/40**

(43) Veröffentlichungstag der Anmeldung:
**10.04.85 Patentblatt 85/15**

(84) Benannte Vertragsstaaten:
**AT BE CH DE FR GB IT LI LU NL SE**

(71) Anmelder: **P + T Patent und Technologie AG**
**Bellerive-Horneggstrasse 4**
**CH-8008 Zürich(CH)**

(72) Erfinder: **Engesser, Felix**
**Baumackerstrasse 58**
**CH-8050 Zürich(CH)**

(74) Vertreter: **EGLI-EUROPEAN PATENT ATTORNEYS**
**Donaustrasse 24**
**D-8000 München 80(DE)**

(54) **Selbstadaptierende Applikationseinrichtung für intermittierte Hochfrequenzpulse zu Kurzwellengeräten für medizinisch-physikalische Therapien.**

(57) Die Applikationseinrichtung besteht aus einer Schaltung (21) für den Behandlungsstrahler (20) und einem Anpassungsnetzwerk (25) mit einer Pickup-Einrichtung ($L_{25'}$) einer Schaltung (FNT) zur Auswertung des Pickup-Signals und zur Steuerung eines Stellmotors (M) und einem Mittel ($C_{25}$) zur Veränderung der Uebertragungsfunktion des Anpassungsnetzwerkes, das mit dem Stellmotor (M) funktionell gekoppelt ist. Der nunmehr sehr einfach mit einem Schwingkreis (21) beschaltete Behandlungsstrahler (20) ist leicht manipulierbar und eignet sich bei adäquater Auslegung zu fokussierender Bestrahlung.

EP 0 136 363 A1

FIG. 2a

Selbstadaptierende Applikationseinrichtung für intermittierte Hochfrequenzpulse zu Kurzwellengeräten für
medizinisch-physikalische Therapien

Die Erfindung liegt auf dem Gebiet der medizinisch-physikalischen Therapie und betrifft eine selbstadaptierende Applikationseinrichtung für intermittierte Hochfrequenzpulse zu Kurzwellengeräten, insbesondere zu solchen
für die nichtthermische Therapie.

Im Kurzwellen- oder Ultrakurzwellenbereich arbeitende Geräte zur wärmefreien therapeutischen Behandlung
eines Patienten sind beispielsweise durch die US-PS
3.181.535 (A.S. Millinowsky, 1965), durch die US-PS 3.299.892 (W.D. Kendall et al, 1967) und durch die DE-OS 21 31 549 (J.J. Pearo, 1972) bekanntgeworden; es sind alles Kurzwellen-Therapiegeräte, welche die Energie in Form von Hochfrequenzpulsen an den Patienten abgeben. Die Aufbereitung solcher Hochfrequenzpulse geschieht meist nach dem folgenden üblichen Schema: In einem Schaltungsteil wird ein hochfrequentes, elektromagnetisches Feld im Megahertzbereich erzeugt und deren Spannung anschliessend in den Kolovoltbereich verstärkt, wobei die in einem anderen Schaltungsteil aufbereitete Pulssequenz beispielsweise zum Schalten eines bzw. des Leistungsverstärkers benützt wird, woraus dann die entsprechenden Hochfrequenzpulse von verhältnismässig hoher Pulsamplitude resultieren. Diese Pulse werden schliesslich auf einen Applikationskopf geführt, aus dem sie dann als gepulste Hochfrequenzfelder auf die zu behandelnde Stelle des Patienten abgestrahlt werden.

Einer Fachperson aus dem Gebiet der Hochfrequenztechnik ist es klar, dass elektromagnetische Felder in diesem, meist oberen Kurzwellenfrequenzbereich nur durch optimale Anpassung mit tolerierbaren, noch in Kauf zu nehmenden Uebertragungsverlusten appliziert werden können.

Soll die Behandlung Erfolg haben, so ist es wichtig, diese Energien in das zu therapierende Gewebe einzubringen, welches Gewebe oftmals in beträchtlicher Körpertiefe liegen kann. Gelingt es dem Fachmann auch, geräteseitig alle Massnahmen zu treffen, die für eine optimale Energieübertragung nötig sind, so ist damit immer noch nicht sichergestellt, dass auch die mit der Therapie betraute Person die wesentlich schwierigere Uebertragungsanpassung des Behandlungskopfes auf Elektrolyt enthaltende Körperstellen des Patienten durchführen kann. Die aus dem Behandlungskopf austretenden elektromagnetischen Felder werden nun nicht mehr, wie dies in der Hochfrequenz üblich ist, nach der Uebertragung der Leistung über ein EM-Feld wieder von galvanischen Leiteranordnungen zur Weiterverwendung aufgenommen, sondern sie dienen lediglich zur physikalischen Beeinflussung des zu behandelnden Körpergewebes.

Die mangelhafte Abstimmung eines Behandlungs- oder Applikationskopfes in bezug auf den zu therapierenden Patienten kann den ganzen Heilerfolg in Frage stellen. Vie Vielfach übertreffen solche Abstimmungsfehler, also Fehler, die unter den Begriff Fehlbedienung einzureihen sind, den möglichen vorgesehenen Energiebereich, der am Apparat selbst, beispielsweise durch Wählknöpfe, einstellbar ist, um ein Mehrfaches. Mit anderen Worten, schrittweise wählbare Behandlungsstärken können so nicht reproduziert werden, weil die Fehlerstreuung aufgrund der vorerwähnten Fehlbedienung solche Behandlungsstärkestufen bei weitem übertrifft. Einstellhilfen, wie beispielsweise induktiv angekoppelte Lampen, deren Leuchtstärke durch Drehen an einem Plattenkondensator maximiert werden muss, sind als ungenügend anzusehen deswegen, weil die Einstellfeinheit in der Gegend des Leuchtstärkenmaximums sehr unempfindlich ist, und weil bei reduzierter Energieabgabe diese induktiv angekoppelte, optische Einstellhilfe in gleichem Masse die Energiereduktion anzeigt, und die beispielsweise schwach glimmende Lampe kaum mehr auf maximale Leuchtstärke eingestellt werden kann. Nicht zuletzt wird oftmals von elektronisch ungeschulten Therapiepersonal die Abgleichoperation einfach vergessen oder ignoriert, so dass die Patienten mit unbekannten Feldstärken therapiert werden, die sich natürlich bei der nächsten Therapiesitzung nicht mehr nachvollziehen lassen. Dies ist auch mit ein Grund, warum auf diesem Gebiet der Kurzwellentherapie Heilerfolge schlecht reproduzierbar sind.

- 5 -

0136363

Es ist Aufgabe der Erfindung, eine Applikationseinrichtung für Ultrakurzwellentherapie zu schaffen, mit
der Fehlbedienungen auf der Applikationsseite durch das
Therapiepersonal ausgeschlossen sind, und damit der Behandlungserfolg wesentlich reproduzierbarer wird. Ferner
ist es Aufgabe der Erfindung, einen vom Gewicht her möglichst
leichten und zudem in seinen elektrischen Bauteilen einfachen und dadurch leicht zu manipulierenden, zur Applikationseinrichtung gehörenden Behandlungskopf zu schaffen,
mit dem es auch möglich ist, durch die schaltungsmässige
Vereinfachung miniaturisierbarer Dimensionierungen eine
fokussierende Bestrahlung durchzuführen.

Beide Aspekte dieser Aufgabe werden durch die
in den Patentansprüchen angegebene Erfindung gelöst. Mit
Hilfe der nachfolgend aufgeführten Figuren wird die Erfindung nun eingehend im einzelnen dargestellt. Es zeigen:

Fig. 1    eine teilweise schematische Darstellung einer
          Applikationseinrichtung für ein Kurzwellentherapiegerät gemäss dem Stand der Technik,

Fig. 2a          eine teilweise schematische Darstellung eines Behandlungskopfes gemäss der Erfindung,


Fig. 2b          eine Kennlinie des in Figur 2a gezeigten Steuermotors in Abhängigkeit der abgestrahlten Hochfrequenzenergie,


Fig. 3a, 3b      schaltungsmässige Details eines Behandlungskopfes gemäss der Erfindung, und


Fig. 4a - 4g     Funktionsdiagramme im Zusammenhang mit den

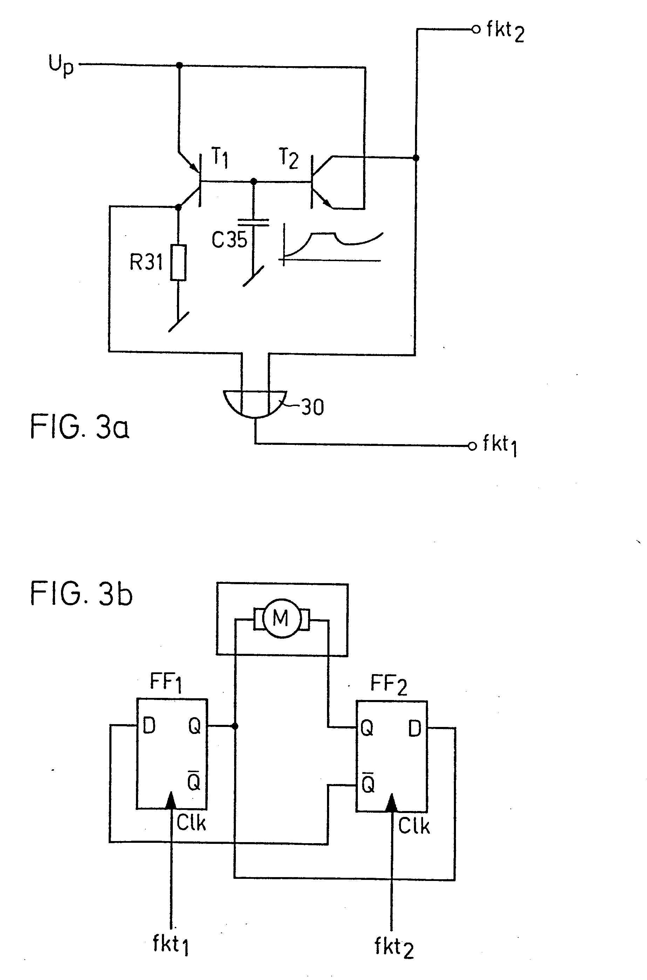

                 Schaltungsteilen nach den Figuren 3a und 3b.


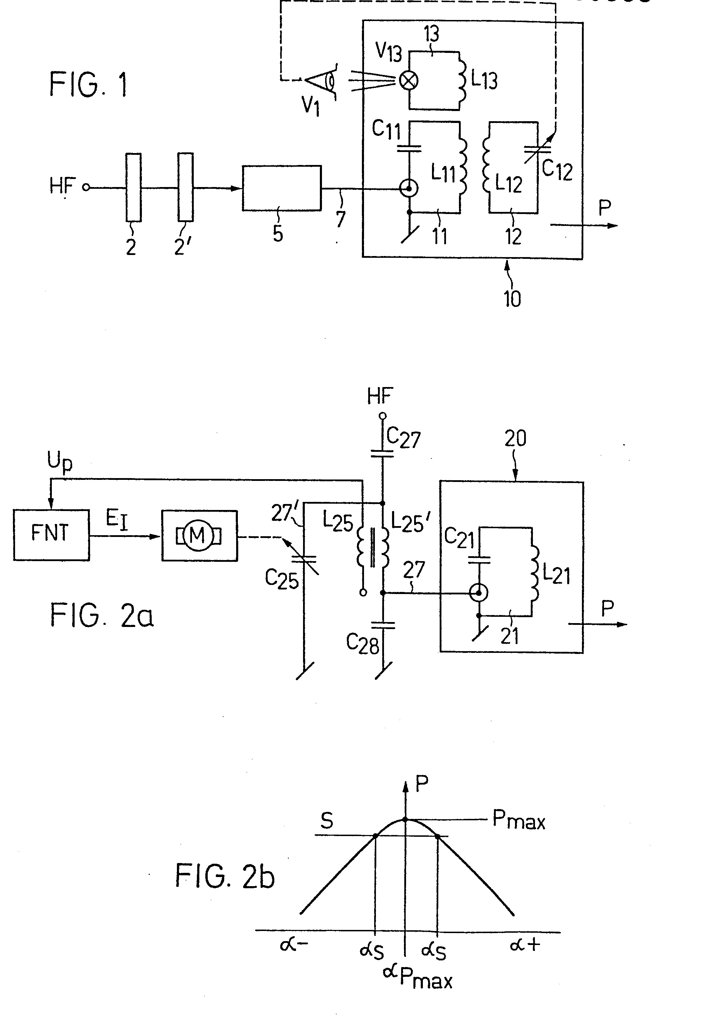

        Figur 1 zeigt in vereinfachter Darstellung eine Applikationseinrichtung für gepulste Kurzwellen gemäss dem Stand der Technik. Von einer HF-Stufe aufbereitet, gelangen einzelne Kurzwellenpulse 2, 2' ... auf ein als Blackbox

5 dargestelltes Uebertragungs- und Anpassungsnetzwerk auf den Behandlungskopf 10 der Applikationseinrichtung. Dieser Behandlungskopf 10 ist mit dem Netzwerk 5 üblicherweise über ein flexibles Koaxialkabel 7 verbunden, dessen Auswahl sich nach dem Wellenwiderstand richtet, der für die Anpassung optimal sein soll. Der Behandlungskopf selbst enthält einen ersten LC-Schwingkreis 11 mit den Elementen $L_{11}$ und $C_{11}$. Daran angekoppelt ist ein zweiter, abstimmbarer LC-Schwingkreis 12 mit den Elementen $L_{12}$ und $C_{12}$ variabel. Während funktionell gesehen der erste Schwingkreis 11 gerätezugewandt und auf das Gerät abgestimmt ist, ist der zweite Schwingkreis patientenzugewandt und muss auf diesen abgestimmt werden.

Für maximale Leistungsübertragung wird der patientenzugewandte Schwingkreis so abgestimmt, dass ein maximaler Energiefluss über die induktive Kopplung $L_{11}$/ $L_{12}$ übertragen wird. Dieser Energiefluss wird von einem weiteren induktiven Kreis 13 mit einer Spule $L_{13}$ gleichsam abgelauscht; der in diesem Kreis fliessende, zum Hauptstrom proportionale Nebenstrom wird beispielsweise durch ein Glühlämpchen $V_{13}$ sichtbar gemacht. Das Auge $V_1$ der Therapieperson nimmt den Leuchtzustand des Lämpchens $V_{13}$ wahr und verstellt von Hand das kapazitive Element $C_{12}$, um das Lämpchen zur maximalen Leuchtstärke zu bringen. Damit wäre auch die Energieübertragung auf den Patienten maximal.

So einfach eine Regeloperation über den Regelkreis Maschine-Mensch-Maschine vielfach abläuft, kann dies
bei mangelhafter Indikation zum Auge zu erheblichen Regelunsicherheiten führen. Fällt zudem der Mensch zeitweise
als Teilglied des Regelkreises aus - dies tritt ein, wenn
die Therapieperson nach ersten Einstellvorgängen den
Patienten verlässt und sich beispielsweise einem weiteren
Patienten zuwendet - so kann sich die Anpassung unbemerkt
massiv verändern. Zeitweilige Nachkontrollen beheben dann
möglicherweise den Fehlzustand der Regelung; es ist doch
eindeutig einsehbar, dass gerade bei dieser Art von Regelanforderung der Mensch recht ungeeignet ist, einen Teil
des Regelkreises zu bilden. Die eingangs geschilderten Mängel sind weitgehend darauf zurückzuführen.

Des weiteren ist gemäss dem Stand der Technik
ausgestalteter Behandlungskopf zwangsweise gewichtig und
relativ gross. Die Bauelemente der Schwingkreise 11 und 12
sind verhältnismässig massive ein- bis zweiwindige Spulen
$L_{11}$ und $L_{12}$ und grossflächige Plattenkondensatoren, insbesondere der variable Kondensator $C_{12}$. Allein das Gewicht
eines solchen Behandlungskopfes erfordert eine relativ
kräftige Halterung am Apparat und daraus resultierende erhebliche Manipulationskräfte, die ergometrisch betrachtet eigentlich vermieden werden sollten.

Demgegenüber zeigt Figur 2a eine Applikationseinrichtung gemäss der Erfindung, die selbsttätig adaptiert und mit einem recht einfachen Schaltungsaufwand auf der Seite des Behandlungskopfes. Der dargestellte Behandlungskopf 20 enthält lediglich einen LC-Schwingkreis 21 mit den Elementen $L_{21}$ und $C_{21}$. Er ist ebenfalls über ein flexibles Koaxialkabel mit dem Grundgerät verbunden. Die Regelung erfolgt ohne Eingriff der Therapieperson.

Gepulste Kurzwellen sind am einen Ende eines Serienschwingkreises mit den Elementen $C_{27}$, $L_{25"}$, $C_{28}$ angelegt. An beiden Enden der Schwingspule $L_{25"}$ werden die HF-Pulse über Leitungen 27 und 27' abgegriffen, einerseits zur Zuleitung auf den patientenzugewandten Schwingkreis 21 und andererseits zu einer variablen Parallelkapazität 25, die von einem Stellmotor M mechanisch veränderbar ist. Eine Pickup-Spule $L_{25}$ tastet den Zustand des variierbaren Schwingkreises ab und liefert ein Regelsignal $U_p$ auf ein Funktionsnetzwerk FNT, das seinerseits über einen Ausgang den Stellmotor mit einem Signal $E_I$ ansteuert.

Figur 2b zeigt eine Funktionskurve des Stellmotors. Auf der Abszisse ist ein Stellwinkel $\alpha$ angegeben, und die Ordinate zeigt die Grösse der Energieübertragung P an. Die Funktions der Energie in Abhängigkeit des Stellwinkels zeigt proportional zur Anpassung des gerätezugewandten Schwingkreises ein Maximum an der Stelle $P_{max}$ bei einem Stellwinkel $\alpha_{P_{max}}$. Ein beispielsweise dargestellter Schwellenwert S in Funktion der dazugehörigen Stellwinkel $\alpha_S$ zeigt einen möglichen Arbeitsbereich des Stellsignales $E_I$. Man sieht daraus, wie die mit dem Stellmotor gekoppelte variable Kapazität $C_{25}$ den gerätezugewandten Schwingkreis auf eine maximale Energieübertragung zum Patienten zugewandten Schwingkreis regelt und dabei ständig den Stellwinkel aussucht, der diese maximale Energieübertragung gewährleistet. In dieser Figur ist nur eine Funktion bei einer Energiestärke dargestellt; man muss jedoch weitere, parametrisch voneinander abhängige Funktionen bei verschiedenen Energiestärken sich vorstellen.

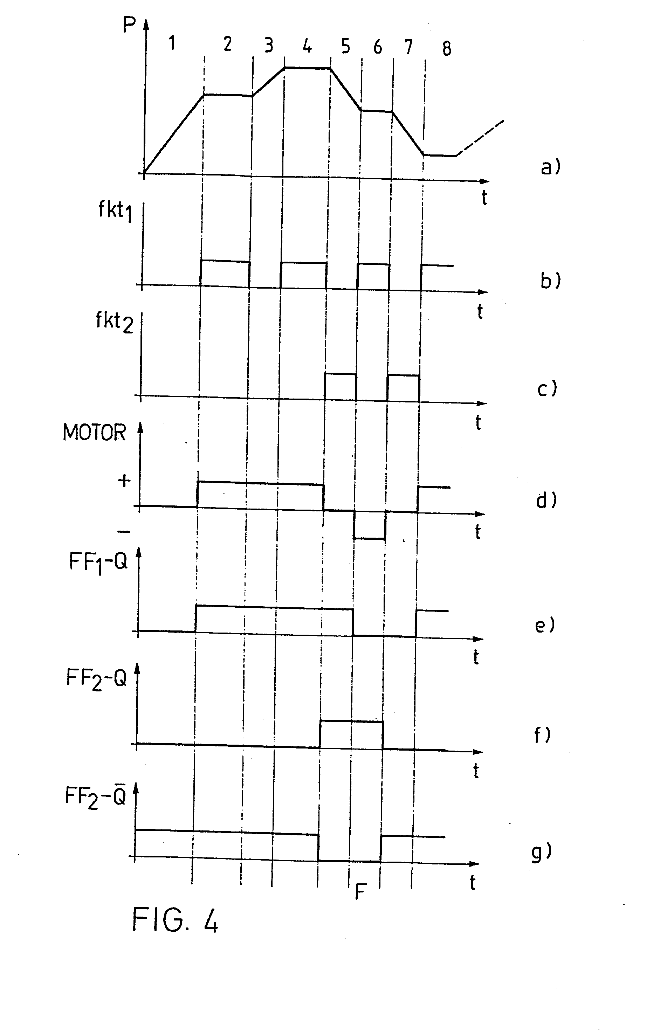

Die Figuren 3a und 3b zeigen wesentliche Schaltungsbeispiele innerhalb der Funktions-Blackbox FNT nach Figur 2a. Funktions- bzw. Signaldiagramme, die die beiden Schaltungsteile gemäss der Figuren 3a und 3b charakterisieren, sind in den Figuren 4a bis 4g dargestellt. Ein Ueberwachungssignal $U_p$ aus der Pickup-Spule $L_{25}$, wird auf die Emitter von zwei Transistoren $T_1$ und $T_2$ geführt. Bei den beiden Transistoren handelt es sich in diesem Beispiel um ein komplementäres Paar. Die Basis der beiden Transistoren ist gemeinsam über eine Kapazität $C_{35}$ am Massepegel angelegt, und von den Kollektoren der beiden Transistoren werden die Signale für beiden Funktionen $fkt_1$ und $fkt_2$ gemäss den Figuren 4b und 4c abgeleitet. Für das Signal $fkt_1$ werden die Kollektorsignale der Transistoren $T_1$ und $T_2$ über ein OR-Gatter 30 verknüpft, während das Kollektorsignal des Transistors $T_2$ direkt als Signal $fkt_2$ verwendet wird. Diese beiden Signale steuern die in Figur 3b gezeigte Schaltung an. Diese Schaltung besteht aus einem Flip-Flop-Paar $FF_1$ und $FF_2$, in diesem Beispiel zwei Einflanken-getriggerte D-Flip-Flop. Zwischen den Q-Ausgängen der beiden Flip-Flop ist die Erregerwicklung des Stellmotors M geschaltet. Der $\overline{Q}$- Ausgang des Flip-Flop $FF_2$ führt auf den D-Eingang des Flip-Flop $FF_1$, während der Q-Ausgang des Flip-Flop $FF_1$ auf den D-Eingang des Flip-Flop $FF_2$ führt. An die Clock-Eingänge Clk der beiden Flip-Flop sind die Signale $fkt_1$ bzw. $fkt_2$ angelegt. Die Signale der Q-Ausgänge beider Flip-Flop sind in den Figuren 4e bis 4g dargestellt, das auf die Erregerwicklung des Stellmotors M wirkende Signal zeigt das Diagramm der Figur 4d, und Figur 4a zeigt zeigt einen möglichen Verlauf der übertragenen Leistung P.

Die Funktion der beiden Schaltungsteile aus der Schaltung FNT lässt sich nun anhand der Diagramme nach den Figuren 4a bis 4g erklären. Beim Start des Gerätes sind beide Flip-Flop $FF_1$ und $FF_2$ in Reset-Stellung, d. h. beide Ausgänge Q, zwischen denen die Erregerwicklung des Stellmotors M gelegt ist, zeigen gleiche Signale und damit gleiches Potential, d. h. der Stellmotor wird nicht betätigt. Dies entspricht auf dem Diagramm von Figur 4a dem Ausgangspunkt Null. Die Energie muss bei eingeschaltetem Gerät von Null weg zwangsweise ansteigen, was durch den ersten Funktionsabschnitt 1 des Diagramms angezeigt ist. Das auf die beiden Transistoren $T_1$ und $T_2$ der Figur 3a wirkende Ueberwachungssignal $U_p$ von der Pickup-Spule $L_{25}$, lässt über die Emitter-Basisstrecke des Transistors $T_1$ die Spannung über den Kondensator $C_{35}$ ansteigen. Bei einem gewissen Spannungspegel am Kondensator $C_{35}$ erscheint am Ausgang des NOR-Gatters 30 eine logische 1, die als Signal $fkt_1$ am Clock-Eingang des Flip-Flop 1 den Q-Ausgang umschaltet. Zwischen den beiden Q-Ausgängen der Flip-Flop

$FF_1$ und $FF_2$ besteht nun eine Potentialdifferenz (Figuren 4e und 4f), die den Stellmotor M in eine der beiden Drehrichtungen bringt (Figur 4d). Nimmt nun, wie dies in Figur 4a im Abschnitt 2 dargestellt ist, der Energiefluss auf den Patienten nicht mehr zu, so dreht der Motor die Abstimmkapazität in der gleichen Richtung weiter. Es ist damit keineswegs festgelegt, dass der Motor in die richtige Drehrichtung arbeitet, doch sei in diesem Beispiel angenommen, dass die Drehrichtung mit der die Kapazität $C_{25}$ nach Figur 2a so verstellt wird, dass die auf den Patienten übertragene Energie zunimmt, was in Figur 4a im Abschnitt 3 durch die Funktionszunahme dargestellt ist. Das Verschwinden des Signals $fkt_1$ am Clock-Eingang des Flip-Flop $FF_1$ verändert den Q-Ausgang des Flip-Flop nicht, wenn dieses ein positiv Flanken-getriggertes Flip-Flop ist, d. h. auch beim Verschwinden dieses Signals läuft der Motor in seiner Drehrichtung unentwegt weiter, was auch ein neuerliches Erscheinen des Signals $fkt_1$ im Abschnitt 4 der Figur 4a nicht ändert. Gegen das Ende des Abschnitts 4 beginnt der Motor über das Signalmaximum P hinauszudrehen und die übertragene Energie beginnt, abzufallen. Dies bewirkt durch Umschalten der Transistoren $T_1$ und $T_2$ das Erscheinen des Signals $fkt_2$ und das gleichzeitige Verschwinden des Signals

fkt$_1$. Die positive Flankentriggerung der Flip-Flops bewirkt nun eine Umstellung des Q-Ausgangs des Flip-Flop
FF$_2$, der Q-Ausgang des Flip-Flop FF$_1$ bleibt dabei unverändert, und die Potentialdifferenz über der Erregerspule
des Stellmotors M wird wieder Null, d. h. der Motor bleibt
stehen, und die Kapazität C$_{21}$ wird nicht mehr weiter verändert. Der Energiefluss über die Schwingkreise auf den
Patienten bleibt wiederum wie in Abschnitt 2 der Figur 4a
auch nun in Abschnitt 6 derselben Figur wieder konstant,
was das Erscheinen des Signals fkt$_1$ bewirkt, welches durch
die positive Flanken-Triggerung das ihm zugeordnete Flip-
Flop FF$_1$ umstellt, d. h. der Q-Ausgang dieses Flip-Flop
geht wieder auf Null, während der Q-Ausgang des Flip-Flop
FF$_2$ unverändert bleibt, was wieder eine Potentialdifferenz
über der Erregerspule des Stellmotors M bewirkt, diesmal
jedoch in der anderen Drehrichtung. Da aber die übertragene
Energie sich abschwächt, müsste der Stellmotor die vorherige Drehrichtung beibehalten; nun ist aber das Gegenteil der
Fall, d. h. der Stellmotor M arbeitet in die falsche Drehrichtung. Nimmt nun, beispielsweise wegen der falschen
Drehrichtung, wie in Figur 4a im Abschnitt 7 gezeigt, die
übertragene Energie weiterhin ab, so erscheint wieder das
Signal fkt$_2$, das ein Umschalten des Flip-Flop FF$_2$ bewirkt,

dessen Q-Ausgang wieder auf logisch Null zurückgeht. Damit ist das Potential über der Erregerspule des Stellmotors M wiederum Null, d. h. der Motor bleibt aus dieser falschen Drehrichtung heraus stehen; dies ist dann die Bedingung, die das Signal $fkt_1$ wieder erscheinen lässt, dessen positive Flanke das Flip-Fop $FF_1$ wiederum triggert und den Q-Ausgang dieses Flip-Flop auf logisch 1 stellt. Die Umstellung der Potentialdifferenz zwischen den beiden Flip-Flop-Ausgängen ändert damit auch die Drehrichtung des Motors, so dass, wie Abschnitt 8 der Figur 4a zeigt, durch diese korrigierte Drehrichtung die übertragene Energie wieder ansteigt.

Das Zusammenwirken der Funktionen der beiden Schaltungen nach den Figuren 3a und 3b mit der Schaltung nach Figur 2a zeigt dann folgende, auf Figur 4a bezogene Charakteristik. Nimmt die übertragene Energie beim Drehen der variablen Kapazität $C_{25}$ zu, so behält der Stellmotor seine Drehrichtung bei. Er behält die eingenommene Drehrichtung auch dann, wenn die übertragene Energie ihren Pegel beibehält, sich also nicht verändert. Sobald die übertragene Energie in ihrer Intensität abfällt, so wird der Stellmotor abgeschaltet und bei darauffolgender, gleichbleibender Energieübertragung nach einer Abnahme der Energie in die andere Drehrichtung umgeschaltet. Nimmt die übertragene Energie weiterhin ab, wird der Motor wieder abgeschaltet und anschliessend durch eine kurze Dauer konstanter Energieübertragung wieder in die andere Drehrichtung übergeschaltet; dies solange, bis die übertragene Energie wieder zunimmt. Aufgrund dieser Charakteristik sucht der Stellmotor in seiner Drehrichtung ununterbrochen das Maximum der Energieübertragung auf, "überfährt" dieses Maximum, wechselt die Drehrichtung, kommt zurück, um in ständigem Wechselspiel zwischen den beiden in Figur 2b angedeuteten Schwellen S die maximale Energieübertragung zu halten. Dieser dynamische Vorgang ist selbstkontrollierend, solange das Gerät auf Therapie geschaltet ist, und die Applikationseinrichtung auf den Patienten einwirkt. Der gerätezugewandte Schwingkreis wird also durch den Stellmotor immer auf maximale Energieübertragung abgestimmt. Es ist dann verhältnismässig einfach, durch weitere Beschaltung des Netzwerkes gemäss der Erfindung optisch oder akustisch wirkende Fehlanzeigefunktionen zu realisieren, beispielsweise für den Hinweis Gerät ausschalten, maximale Energieübertragung zu gering bei Vergrösserung des Abstandes Patient-Behandlungskopf, etc.

PATENTANSPRÜCHE

1.  Applikationseinrichtung für intermittierte Hochfre-
    quenzpulse zu Kurzwellengeräten für medizinisch-
    physikalische Therapien, bestehend aus einem Behandlungsstrahler und einem oder mehreren Anpassungsnetzwerken für die Leistungsübertragung, gekennzeichnet
    durch einen Behandlungsstrahler (20) mit einem
    Schwingkreis (21) und ohne Mittel zur Anpassung der
    Leistungsübertragung sowie einem vom Behandlungsstrahler gesonderten Anpassungsnetzwerk (35).

2.  Applikationseinrichtung nach Anspruch 1, dadurch gekennzeichnet, dass das Anpassungsnetzwerk (25) eine
    Pickup-Einrichtung ($L_{25}$,) zur Ueberwachung der übertragenen Leistung aufweist.

3. Applikationseinrichtung nach den Ansprüchen 1 und 2, dadurch gekennzeichnet, dass im Anpassungsnetzwerk (25) Mittel (FNT) zur Auswertung des Pickup-Signals, Mittel ($C_{25}$) zur Veränderung der Uebertragungsfunktion des Anpassungsnetzwerkes (25) und Mittel (M) zum Einstellen des Veränderungsmittels ($C_{25}$) vorhanden sind.

4. Applikationseinrichtung nach Anspruch 3, dadurch gekennzeichnet, dass die Pickup-Einrichtung ($L_{25'}$) an die Emitter zweier komplementärer Transistoren ($T_1$, $T_2$) angeschlossen ist, deren Basis gemeinsam an einem kapazitiv wirkenden Schaltelement ($C_{35}$) anliegt und deren Kollektoren auf die Eingänge eines logischen Schaltelements (30) führen.

5. Applikationseinrichtung nach Anspruch 4, dadurch gekennzeichnet, dass die Kollektoren der komplementären Transistoren ($T_1$, $T_3$) mit dem Takteingang (Clk) eines ersten und eines zweiten D-Flip-Flop ($FF_1$, $FF_2$) verbunden sind, und der Q-Ausgang eines ersten Flip-Flop mit dem D-Eingang eines zweiten Flip-Flop und der $\overline{Q}$-Ausgang des zweiten Flip-Flop mit dem D-Eingang des ersten Flip-Flop verbunden sind, und dass in Serie zu den beiden Q-Ausgängen des ersten und zweiten Flip-Flop die Erregerwicklung eines Elektromotors (M) geschaltet ist.

FIG. 1

1/3

0136363

FIG. 2a

FIG. 2b

FIG. 3a

FIG. 3b

0136363

FIG. 4

**0136363**
Nummer der Anmeldung

Europäisches
Patentamt

**EUROPÄISCHER RECHERCHENBERICHT**

EP  83 10 8821

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-3 543 762 (KENDALL)<br>* Spalte 2, Zeilen 6-19, 61-68; Spalte 3, Zeilen 19-25 *<br><br>--- | 1-3 | A 61 N   1/40 |
| Y | US-A-3 952 751 (KENDALL)<br>* Spalte 2, Zeilen 23-32; Spalte 4, Zeilen 17-25 *<br><br>--- | 1 | |
| Y | US-A-4 210 152 (BERRY)<br>* Spalte 6, Zeile 47 - Spalte 7, Zeile 13 *<br><br>--- | 1,3 | |
| A | US-A-3 513 851 (SMITH)<br>* Spalte 2, Zeilen 32-36 *<br><br>--- | 1 | |
| A | FR-A-1 369 150 (VARTA)<br>* Seite 2, Abschnitt 3 *<br><br>--- | 4 | **RECHERCHIERTE SACHGEBIETE (Int. Cl. ³)** |
| A | FR-A-2 254 898 (ATELIERS DE SECHERON)<br>* Seite 7, Zeile 29 - Seite 8, Zeile 14 *<br><br>--- | 4 | A 61 N<br>H 05 B<br>H 03 J<br>G 01 R |
| E | GB-A-2 126 098 (METRONEX)<br>* Seite 1, Zeilen 97-99; Seite 2, Zeilen 50-83 *<br><br>----- | 1-3 | |

Der vorliegende Recherchenbericht wurde für alle Patentansprüche erstellt.

| Recherchenort<br>DEN HAAG | Abschlußdatum der Recherche<br>07-05-1984 | Prüfer<br>SIMON J.J.E. |
|---|---|---|